# EUROPÄISCHE PATENTANMELDUNG

(11) **EP 4 703 343 A1**
(43) Veröffentlichungstag der Anmeldung: **04.03.2026**
(21) Anmeldenummer: 24197653.9
(22) Anmeldetag: 30.08.2024
(51) Int. Cl.: C07C 29/151, C07C 31/04

(54) **VERFAHREN ZUR HERSTELLUNG VON METHANOL**

(71) Anmelder: L'AIR LIQUIDE, SOCIETE ANONYME POUR L'ETUDE ET L'EXPLOITATION DES PROCEDES GEORGES CLAUDE, 75007 Paris (FR)
(72) Erfinder: Janko, Lutz, 60439 Frankfurt am Main (DE); Peña, Vincent, 60439 Frankfurt am Main (DE); Blickhan, Nina, 60439 Frankfurt am Main (DE)
(74) Vertreter: Air Liquide

(57) **Zusammenfassung**

Ein Verfahren zur Herstellung von Methanol aus einem wasserstoffreichen und einem kohlendioxidreichen Eduktgasstrom. Erfindungsgemäß wird ein in einem Niederdruckabscheider erhaltener Entspannungsgasstrom, welcher Wasserstoff und Kohlendioxid enthält, zu einem der beiden Eduktgasströme zurückgeführt, oder der Entspannungsgasstrom wird auf zwei Teilströme aufgeteilt, und die jeweiligen Teilströme werden zu den jeweiligen Eduktgasströmen zurückgeführt. Die Zusammenführung dieser Ströme erfolgt vor Verdichtung auf Synthesedruck und vor der Bildung des eigentlichen Synthesegasstroms.

## Beschreibung

Die Erfindung betrifft ein Verfahren zur Herstellung von Methanol aus einem ersten wasserstoffreichen und einem zweiten kohlendioxidreichen Eduktgasstrom, sowie aus einem wasserstoff- und kohlendioxidhaltigen Entspannungsgasstrom.

Methanol wird im großtechnischen Maßstab durch Umsetzung von Synthesegas an einem geeigneten Feststoffkatalysator bei Synthesedrücken von bis zu 100 bar hergestellt. Synthesegas ist üblicherweise ein Gemisch aus vorwiegend Wasserstoff (H₂), Kohlenmonoxid (CO) sowie Kohlendioxid (CO₂). Kohlenmonoxid sowie Kohlendioxid werden dabei häufig unter dem Begriff "Kohlenoxide" oder "Kohlenstoffoxide" zusammengefasst. Dabei laufen an einem festen Methanolsynthese-Katalysator vornehmlich die beiden folgenden Gleichgewichtsreaktionen (1) und (2) nebeneinander ab.

(1) CO₂ + 3 H₂ # CH₃OH + H₂O

(2) CO + 2 H₂ # CHsOH

Methanol kann auch aus Synthesegas hergestellt werden, welches arm an oder sogar frei von Kohlenmonoxid ist. Entsprechend kommt dann vorwiegend Reaktionsgleichung (1) bei der Methanolsynthese zur Geltung. Die Herstellung dieses sogenannten kohlendioxidbasierten Methanols spielt insbesondere bei Prozessen eine Rolle, welche ohne oder zumindest mit möglichst wenigen fossilen Energieträgern oder fossilen Einsatzstoffen realisiert werden sollen und möglichst wenige Treibhausgasemissionen erzeugen sollen.

So kann kohlendioxidbasiertes Methanol beispielsweise auf der Basis von durch Verbrennung eines fossilen Einsatzstoffes erzeugtem Kohlendioxid und regenerativ erzeugtem Wasserstoff hergestellt werden. Der regenerativ erzeugte Wasserstoff wird vorzugsweise durch Elektrolyse von Wasser auf Basis regenerativ erzeugten Stroms hergestellt.

Dem eigentlichen Methanolsynthese-Reaktor ist üblicherweise eine Kaskade aus mindestens zwei seriell angeordneten Abscheidern nachgeschaltet. In einem ersten Hochdruckabscheider wird Rohmethanol als Flüssigkeit abgeschieden und zumindest ein Teil der verbleibenden gasförmige Phase, welche nicht reagiertes Synthesegas enthält, zum Einlass des Methanolsynthese-Reaktors zurückgeführt. Das aus dem Hochdruckabscheider ausgeleitete Rohmethanol wird nach Druckabsenkung in einen Niederdruckabscheider eingeleitet. Durch die Druckabsenkung gast weiteres nicht umgesetztes Synthesegas aus dem Rohmethanol aus. Dieses als Entspannungsgas bezeichnete Gemisch wird üblicherweise verbrannt oder einer Verwendung als Brennstoff zugeführt. Die Menge dieses Expansionsgases ist so gering und dessen Druck so niedrig, dass es sich in den meisten Fällen nicht lohnt, dieses zum Synthesegas-Einsatzstrom zurückzuführen. Da der Synthesegas-Einsatzstrom üblicherweise einen höheren Druck aufweist als der aus dem Niederdruckabscheider ausgeleitete Entspannungsgasstrom, müsste der Entspannungsgasstrom zunächst durch einen dedizierten Kompressor verdichtet werden, um diesen mit dem Synthesegas-Einsatzstrom zusammenführen zu können.

Bei der kohlendioxidbasierten Methanolsynthese weist der Synthesegas-Einsatzstrom häufig einen niedrigeren Druck auf als der Synthesegas-Einsatzstrom einer konventionellen Methanolsynthese. In WO 2021/110565 wird daher vorgeschlagen, den Entspannungsgasstrom mit dem Synthesegas-Frischgasstrom zu mischen und anschließend auf Synthesedruck zu verdichten, um den Entspannungsgasstrom einer mit weniger Kohlendioxidemissionen verbundenen Verwertung zuzuführen.

Bei der kohlendioxidbasierten Methanolsynthese werden üblicherweise zwei Eduktgasströme bereitgestellt, was diese Art der Synthese von einer konventionellen Methanolsynthese unterscheidet. Bei der konventionellen Methanolsynthese wird üblicherweise ein einziger Synthesegas-Einsatzstrom, welcher beispielsweise aus einem Reformer oder Vergaser ausgeleitet wird, auf Synthesedruck verdichtet und zu Methanol umgesetzt. Bei der kohlendioxidbasierten Methanolsynthese hingegen weisen die beiden Eduktgasströme, der wasserstoffreiche und der kohlendioxidreiche Eduktgasstrom, häufig unterschiedlich hohe Drücke auf. Diese beiden Ströme werden deshalb häufig getrennt voneinander auf Synthesedruck verdichtet und erst anschließend für die Umsetzung im Methanolsynthese-Reaktor zusammengeführt. Ein Mischen des Entspannungsgasstroms mit dem verdichteten Synthesegas-Strom ist dann aus oben genannten Gründen entweder nicht möglich oder wirtschaftlich und technisch nicht sinnvoll, da für den relativ geringfügigen Entspannungsgasstrom ein dedizierter Kompressor benötigt würde.

Aufgabe der vorliegenden Erfindung ist es daher, ein Verfahren vorzuschlagen, welches vorgenannten Nachteil zumindest teilweise überwindet.

Gemäß einem Aspekt der Erfindung wird ein Verfahren zur Herstellung von Methanol vorgeschlagen, wobei das Verfahren folgende Verfahrensschritte umfasst:
(a) Bereitstellen eines ersten Eduktgasstroms, welcher reich an Wasserstoff (H₂) ist;
(b) Bereitstellen eines zweiten Eduktgasstroms, welcher reich an Kohlendioxid (CO₂) ist;
(c) Bereitstellen eines Entspannungsgasstroms, welcher Wasserstoff und Kohlendioxid enthält;
(d1) Zusammenführen des Entspannungsgasstroms mit dem ersten Eduktgasstrom vor Verdichtung auf Synthesedruck zum Bilden eines ersten Mischgasstroms, und Bilden eines Synthesegasstroms durch Zusammenführen des ersten Mischgasstroms mit dem zweiten Eduktgasstrom, oder
(d2) Zusammenführen des Entspannungsgasstroms mit dem zweiten Eduktgasstrom vor Verdichtung auf Synthesedruck zum Bilden eines zweiten Mischgasstroms, und Bilden eines Synthesegasstroms durch Zusammenführen des ersten Eduktgasstroms mit dem zweiten Mischgasstroms, oder
(d3) Aufteilen des Entspannungsgasstroms in einen ersten Teilstrom und einen zweiten Teilstrom, und Zusammenführen des ersten Teilstroms mit dem ersten Eduktgasstrom vor Verdichtung auf Synthesedruck zum Bilden eines dritten Mischgasstroms, und Zusammenführen des zweiten Teilstroms mit dem zweiten Eduktgasstrom vor Verdichtung auf Synthesedruck zum Bilden eines vierten Mischgasstroms, und Bilden eines Synthesegasstroms durch Zusammenführen des dritten Mischgasstroms mit dem vierten Mischgasstrom;
(e) Umsetzen des Synthesegasstroms bei Synthesedruck in einer Reaktionsvorrichtung an einem festen Methanolsynthese-Katalysator zu einem methanolhaltigen Produktgasstrom;
(f) Abtrennen von Rohmethanol aus dem Produktgasstrom in einem ersten Abscheider, wobei ein erster Rohmethanolstrom als flüssige Phase und ein Strom aus nicht reagiertem Synthesegas als gasförmige Phase erhalten werden, und wobei zumindest ein Teil des Stroms aus nicht reagiertem Synthesegas zu einem Einlass der Reaktionsvorrichtung zurückgeführt wird, und der erste Rohmethanolstrom aus dem ersten Abscheider ausgeleitet wird;
(g) Einleiten des ersten Rohmethanolstroms in einen zweiten Abscheider, wobei der Druck im zweiten Abscheider gegenüber dem Druck im ersten Abscheider vermindert ist, wodurch im zweiten Abscheider ein zweiter Rohmethanolstrom als flüssige Phase und der Entspannungsgasstrom als gasförmige Phase erhalten werden, wobei der zweite Rohmethanolstrom und der Entspannungsgasstrom aus dem zweiten Abscheider ausgeleitet werden, und wobei der Entspannungsgasstrom durch das Ausleiten aus dem zweiten Abscheider bereitgestellt wird.

Im Sinne des erfindungsgemäßen Verfahrens wird der Entspannungsgasstrom mit dem ersten wasserstoffreichen Eduktgasstrom zusammengeführt oder mit dem zweiten kohlendioxidreichen Eduktgasstrom zusammengeführt. Alternativ dazu wird der Entspannungsgasstrom auf zwei Teilströme aufgeteilt und der jeweilige Teilstrom mit dem wasserstoffreichen Eduktgasstrom und mit dem zweiten kohlendioxidreichen Eduktgasstrom zusammengeführt. Die Zusammenführung der jeweiligen Ströme erfolgt vor Verdichtung auf Synthesedruck. Anschließend wird durch Zusammenführen der jeweiligen Ströme der eigentliche Synthesegasstrom gebildet.

In jedem Fall findet die Verdichtung auf Synthesedruck gemäß Merkmal (e) erst statt, nachdem der jeweilige Eduktgasstrom oder die Eduktgasströme mit dem Entspannungsgasstrom zusammengeführt wurde(n). Der Ausdruck "Synthesedruck" bedeutet in diesem Zusammenhang, dass das Synthesegas auf einen für die Methanolsynthese geeigneten Druck verdichtet wird. Dabei kann die Verdichtung vor oder nach dem Zusammenführen der entsprechenden Ströme (Eduktgasströme, Mischgasströme) zum Synthesegasstrom erfolgen.

Gemäß einem Beispiel wird der erste Eduktgasstrom durch Elektrolyse von Wasser oder einem anderen geeigneten Edukt gebildet.

Gemäß einem weiteren Beispiel stammt der zweite Eduktgasstrom aus einer Kohlendioxid-Abscheidanlage, wobei das Kohlendioxid in dieser Abscheidanlage durch Absorption und/oder Adsorption zunächst gebunden und anschließend durch Desorption wieder freigesetzt wird. Alternativ dazu kann das Kohlendioxid in der Abscheideanlage durch kryogene Verflüssigung von Kohlendioxid mit optional nachgeschalteter Destillation erhalten werden.

Die Reaktionsvorrichtung kann mehrere parallel oder seriell geschaltete Reaktoren mit Methanolsynthese-Katalysator umfassen. Entscheidend ist lediglich, dass stromab der Methanolsynthese ein methanolhaltiger Produktgasstrom erhalten wird, aus dem durch entsprechende technische Maßnahmen wie Abkühlung, Kondensation und Abscheidung ein flüssiger Rohmethanolstrom erhalten wird. Dieser flüssige Rohmethanolstrom wird zunächst einem ersten Abscheider zugeführt, der auch als Hochdruckabscheider bezeichnet wird.

Unter dem Begriff "Rohmethanol" wird ein Gemisch verstanden, welches zumindest Methanol (CHsOH), Wasser und nicht zu vermeidenden Verunreinigungen wie höhere Alkohole und Ketone enthält.

Die Umsetzung des Synthesegases zu Methanol und Wasser in der Reaktionsvorrichtung erfolgt bei Synthesedruck, beispielsweise einem Druck von 40 bar bis 100 bar, vorzugsweise 75 bar bis 90 bar. Typische bei der Methanolsynthese verwendete Druckbereiche sind dem Fachmann hinlänglich bekannt.

Im ersten Abscheider oder Hochdruckabscheider findet eine Gas-Flüssigkeits-Trennung statt, wodurch ein Strom aus nicht reagiertem Synthesegas und ein erster Rohmethanolstrom erhalten werden. Der Strom aus nicht reagiertem Synthesegas kann auch als Recycle-Strom oder Kreislaufgas-Strom bezeichnet werden. Dieser Strom wird zu einem Einlass der Reaktionsvorrichtung zurückgeführt. Weist die Reaktionsvorrichtung mehrere Reaktoren auf, kann es sich um jeden beliebigen Einlass eines Reaktors handeln. Entscheidend ist lediglich, dass das nicht reagierte Synthesegas erneut der Umsetzung an dem Methanolsynthese-Katalysator unterzogen wird.

Im zweiten Abscheider oder Niederdruckabscheider findet eine weitere Gas-Flüssigkeits-Trennung statt. Der Druck im zweiten Abscheider ist gegenüber dem ersten Abscheider vermindert. Der Druck des dem zweiten Abscheiders zugeführten Rohmethanolstroms kann beispielsweise durch ein Druckminderventil reduziert werden. Durch die Druckminderung wird im zweiten Abscheider der Entspannungsgasstrom erhalten. Dieser wird zum ersten Eduktgasstrom zurückgeführt, oder zum zweiten Eduktgasstrom zurückgeführt, oder in zwei Teilströme aufgeteilt und jeweils ein Teilstrom zum ersten Eduktgasstrom und zum zweiten Eduktgasstrom zurückgeführt. Der Entspannungsgasstrom wird somit nicht zu einem bereits vorgemischten Synthesegasstrom, gebildet aus erstem und zweitem Eduktgasstrom, zurückgeführt.

Dadurch kann das Verfahren flexibler ausgestaltet werden. Insbesondere kann darauf basierend entschieden werden, ob die beiden Haupteduktgasströme (der wasserstoffreiche und der kohlendioxidreiche Strom) getrennt voneinander verdichtet werden, oder ob diese gemeinsam verdichtet werden. Im ersten Fall ist die Nutzung des Entspannungsgasstroms als Teil des Einsatzgasstroms nur möglich, wenn dieser nicht dem bereits vorgemischten Synthesegasstrom zugeführt wird. Bei getrennter Verdichtung ist der Druck stromab der Verdichter nach Zusammenführen der Ströme zu hoch, um den Entspannungsgasstrom an dieser Stelle zuführen zu können.

Eine bevorzugte Ausführungsform des erfindungsgemäßen Verfahrens ist dadurch gekennzeichnet, dass
(h1) der erste Mischgasstrom und der zweite Eduktgasstrom getrennt voneinander auf Synthesedruck verdichtet werden, und nach Verdichtung auf Synthesedruck zusammengeführt werden, um den Synthesegasstrom zu bilden, oder
(h2) dass der erste Eduktgasstrom und der zweite Mischgasstrom getrennt voneinander auf Synthesedruck verdichtet werden, und nach Verdichtung auf Synthesedruck zusammengeführt werden, um den Synthesegasstrom zu bilden, oder
(h3) dass der dritte Mischgasstrom und der vierte Mischgasstrom getrennt voneinander auf Synthesedruck verdichtet werden, und nach Verdichtung auf Synthesedruck zusammengeführt werden, um den Synthesegasstrom zu bilden.

Ein getrennte Verdichtung der beiden Haupteduktgasströme ist insbesondere von Vorteil, wenn beide Ströme mit unterschiedlichen Drücken bereitgestellt werden. Oft ist dann zumindest eine Vorverdichtung eines der beiden Ströme erforderlich, oder unterschiedliche Verdichter oder mehrere seriell angeordnete Verdichter je Eduktgasseite sind erforderlich.

Ferner ist häufig die getrennte Zwischenspeicherung beider Haupteduktgasströme erforderlich. Die Zwischenspeicherung von Wasserstoff ist beispielsweise dann notwendig, wenn dieser Wasserstoff durch Elektrolyse mit Strom aus einer erneuerbaren Energiequelle erzeugt wird. Eine gemeinsame Speicherung ist aufgrund der Möglichkeit der Kondensation von Kohlendioxid in der Regel nicht möglich. In diesem Fall ist daher eine getrennte Verdichtung beider Ströme erforderlich, beispielsweise um einen bestimmten Mindestdruck in dem jeweiligen Speichertank zu ermöglichen, oder um den Speichertank in einem größeren Druckbereich betreiben zu können und dementsprechend eine größere Menge an eingespeichertem Gas nutzbar zu machen.

Entsprechend der Bezeichnung der Alternativen der Prozessschritte d1 bis d3 sind die Alternativen der Prozessschritte h1 bis h3 entsprechend zuzuordnen. Das heißt d1 ist kombinierbar mit h1, d2 ist kombinierbar mit h2, und d3 ist kombinierbar mit h3.

Ein weitere alternative Ausführungsform des erfindungsgemäßen Verfahrens ist dadurch gekennzeichnet, dass
(i1) der erste Mischgasstrom und der zweite Eduktgasstrom zusammengeführt werden um den Synthesegasstrom zu bilden, und der gebildete Synthesegasstrom anschließend auf Synthesedruck verdichtet wird, oder
(i2) dass der erste Eduktgasstrom und der zweite Mischgasstrom zusammengeführt werden um den Synthesegasstrom zu bilden, und der gebildete Synthesegasstrom anschließend auf Synthesedruck verdichtet wird, oder
(i3) dass der dritte Mischgasstrom und der vierte Mischgasstrom zusammengeführt werden um den Synthesegasstrom zu bilden, und der gebildete Synthesegasstrom anschließend auf Synthesedruck verdichtet wird.

Entsprechend der Bezeichnung der Alternativen der Prozessschritte d1 bis d3 sind die Alternativen der Prozessschritte i1 bis i3 entsprechend zuzuordnen. Das heißt d1 ist kombinierbar mit i1, d2 ist kombinierbar mit i2, und d3 ist kombinierbar mit i3.

Eine weitere bevorzugte Ausführungsform des erfindungsgemäßen Verfahrens ist dadurch gekennzeichnet, dass der erste Eduktgasstrom und der zweite Eduktgasstrom unterschiedliche Drücke aufweisen, und dass der Entspannungsgasstrom mit demjenigen Eduktgasstrom zusammengeführt wird, welcher den niedrigeren Druck gegenüber dem anderen Eduktgasstrom aufweist.

Wie bereits vorher erwähnt weisen der erste Eduktgasstrom und der zweite Eduktgasstrom je nach der Art der genutzten Produktionsquelle in der Regel unterschiedlich hohe Drücke auf. Dabei ist es von Vorteil, den Entspannungsgasstrom vor Verdichtung auf Synthesedruck demjenigen Eduktgasstrom zuzuführen, welcher den niedrigeren Druck aufweist. Je niedriger der Druck des Eduktgasstroms, desto niedriger kann auch der Druck im zweiten Abscheider gewählt werden. Je niedriger der Druck im zweiten Abscheider ist, desto höher ist die Menge an Entspannungsgas, welches im zweiten Abscheider aus dem zweiten Rohmethanolstrom ausgast und nach Rückführung zu zumindest einem der Eduktgasströme für die Methanolsynthese genutzt werden kann.

Gemäß dieser Ausführungsform ist weiterhin bevorzugt, dass der Entspannungsgasstrom einen höheren Druck aufweist als der Eduktgasstrom mit dem gegenüber dem anderen Eduktgasstrom niedrigeren Druck, und dass der Entspannungsgasstrom vor dem Zusammenführen mit dem Eduktgasstrom, welcher gegenüber dem anderen Eduktgasstrom einen niedrigeren Druck aufweist, nicht verdichtet wird.

Gemäß der beiden vorgenannten bevorzugten Ausführungsformen wird der Entspannungsgasstrom nicht in einen ersten Teilstrom und einen zweiten Teilstrom aufgeteilt, und es wird kein dritter und vierter Mischgasstrom gebildet.

Eine bevorzugte Ausführungsform des erfindungsgemäßen Verfahrens ist dadurch gekennzeichnet, dass der zweite Eduktgasstrom, der zweite Mischgasstrom oder der vierte Mischgasstrom vor dem Verdichten auf Synthesedruck einem Reinigungsverfahren unterzogen wird, insbesondere einem Reinigungsverfahren zur Entfernung von schwefelhaltigen Verbindungen oder zur Entfernung von Sauerstoff unterzogen wird.

Dadurch werden Substanzen aus den vorgenannten Ströme entfernt, welche Katalysatorgifte für den Methanolsynthese-Katalysator darstellen können oder anderweitig störend sind.

Dabei ist weiter bevorzugt, dass der im Entspannungsgas enthaltene Wasserstoff zur Reduktion von im zweiten Eduktgasstrom enthaltenen Verunreinigungen genutzt wird.

Beispielsweise kann der im Entspannungsgas enthaltene Wasserstoff zum Hydrieren von im zweiten Eduktgasstrom enthaltenen Schwefelverbindungen genutzt werden. In diesem Fall wird somit der zweite Mischgasstrom oder der vierte Mischgasstrom dem entsprechenden Reinigungsverfahren zugeführt, und der im Entspannungsgasstrom enthaltene Wasserstoff wird zum Hydrieren von Schwefelverbindungen genutzt, beispielsweise im Rahmen eines Hydrodesulfurierungsverfahrens, bei dem substanzgebundener Schwefel letztlich in Form von Schwefelwasserstoff (H₂S) freigesetzt und dieser daraufhin adsorptiv aus dem zu reinigenden Strom abgeschieden wird. Dadurch kann die Menge an zu importierendem Wasserstoff oder an zu nutzendem Eduktwasserstoff reduziert werden, oder es wird sogar kein zusätzlicher Wasserstoff benötigt.

Gemäß einem weiteren Beispiel kann der im Entspannungsgas enthaltene Wasserstoff zur katalytischen Umsetzung von Sauerstoff unter Bildung von Wasser genutzt werden. Das Wasser kann anschließend durch Adsorption an einem geeigneten Adsorbens (zum Beispiel einem Molekularsieb) aus dem jeweiligen kohlendioxidreichen Strom entfernt werden.

Eine bevorzugte Ausführungsform des erfindungsgemäßen Verfahrens ist dadurch gekennzeichnet, dass
- der erste Eduktgasstrom mindestens 50 mol-% Wasserstoff enthält, vorzugsweise mindestens 70 mol-% Wasserstoff enthält, weiter bevorzugt mindestens 80 mol-% Wasserstoff enthält, weiter bevorzugt mindestens 90 mol-% Wasserstoff enthält, weiter bevorzugt mindestens 95 mol-% Wasserstoff enthält, weiter bevorzugt mindestens 99 mol-% Wasserstoff enthält, und
- der zweite Eduktgasstrom mindestens 50 mol-% Kohlendioxid enthält, vorzugsweise mindestens 70 mol-% Kohlendioxid enthält, weiter bevorzugt mindestens 80 mol-% Kohlendioxid enthält, weiter bevorzugt mindestens 90 mol-% Kohlendioxid enthält, weiter bevorzugt mindestens 95 mol-% Kohlendioxid enthält, weiter bevorzugt mindestens 99 mol-% Kohlendioxid enthält.

Bei weiteren im ersten und/oder zweiten Eduktgasstrom enthaltenen Bestandteilen kann es sich beispielsweise um unter den Bedingungen der Methanolsynthese inerte Bestandteile handeln. Insbesondere in kohlendioxidreichen Abgasen aus Industrieprozessen kann der Anteil an inerten Bestandteilen, beispielsweise an Stickstoff, besonders hoch sein.

Gemäß einer weiteren Ausführungsform ist der erste Eduktgasstrom ein reiner Wasserstoffstrom und der zweite Eduktgasstrom ein reiner Kohlendioxidstrom.

Weder der erste noch der zweite Eduktgasstrom sind als Synthesegas-Strom aufzufassen, auch nicht nach Zuführen des Entspannungsgasstroms. Das heißt, keiner der vorgenannten Ströme (erster Eduktgasstrom, zweiter Eduktgasstrom, erster bis vierter Mischgasstrom) ist als solcher für eine Methanolsynthese geeignet. Ein für die Methanolsynthese geeigneter (Synthesegas-)Strom wird erst durch Zusammenführen der entsprechenden Ströme erhalten.

Eine weitere bevorzugte Ausführungsform des erfindungsgemäßen Verfahrens ist dadurch gekennzeichnet, dass
- der erste Eduktgasstrom einen Druck von 1 bar bis 90 bar aufweist, vorzugsweise einen Druck von 1 bis 50 bar aufweist, weiter bevorzugt einen Druck von 1 bis 40 bar aufweist, und
- der zweite Eduktgasstrom einen Druck von 1 bar bis 90 bar aufweist, vorzugweise einen Druck von 1 bar bis 50 bar aufweist, weiter bevorzugt einen Druck von 1 bis 40 bar aufweist.

Eine weitere bevorzugte Ausführungsform des erfindungsgemäßen Verfahrens ist dadurch gekennzeichnet, dass der erste Abscheider einen Behälterinnendruck von 40 bar bis 100 bar aufweist, vorzugsweise einen Behälterinnendruck von 75 bar bis 90 bar aufweist.

Eine weitere bevorzugte Ausführungsform des erfindungsgemäßen Verfahrens ist dadurch gekennzeichnet, dass der zweite Abscheider einen Behälterinnendruck von 1 bar bis 50 bar aufweist, vorzugsweise einen Behälterinnendruck von 5 bar bis 35 bar aufweist.

Eine weitere bevorzugte Ausführungsform des erfindungsgemäßen Verfahrens ist dadurch gekennzeichnet, dass der gemäß Schritt (g) aus dem zweiten Abscheider ausgeleitete Entspannungsgasstrom einem Gasanreicherungsverfahren unterzogen wird, wodurch ein Entspannungsgasstrom erhalten wird, in welchem die Wasserstoffkonzentration und/oder die Kohlendioxidkonzentration gegenüber dem aus dem zweiten Abscheider ausgeleiteten Entspannungsgasstrom erhöht ist, und wobei der so erhaltene an Wasserstoff und/oder Kohlendioxid angereicherte Entspannungsgasstrom als Entspannungsgasstrom gemäß Schritt (c) bereitgestellt wird.

Beispiele für geeignete Gasanreicherungsverfahren sind die Trennung der Gaskomponenten durch Wechseldruckadsorption (PSA) oder Membrantrennverfahren, oder eine Kombination daraus.

Eine weitere bevorzugte Ausführungsform des erfindungsgemäßen Verfahrens ist dadurch gekennzeichnet, dass von dem Strom aus nicht reagiertem Synthesegas ein Teilstrom als Purge-Gas-Strom abgezweigt wird, und der Purge-Gas-Strom einer Wasserstoff-Rückgewinnungseinheit zur Erzeugung eines wasserstoffreichen Stroms zugeführt wird, und wobei zumindest ein Strom aus der Gruppe erster Eduktgasstrom, zweiter Eduktgasstrom, erster Mischgasstrom, zweiter Mischgasstrom, dritter Mischgasstrom, vierter Mischgasstrom, Entspannungsgasstrom vor Verdichten auf Synthesedruck durch Zuführen von Wasserstoff aus dem wasserstoffreichen Strom an Wasserstoff angereichert wird.

Geeignete Wasserstoffrückgewinnungseinheiten enthalten beispielsweise Vorrichtungen zur Trennung von Gaskomponenten durch Wechseldruckadsorption (PSA) oder Membrantrennverfahren, oder eine Kombination daraus.

Der wasserstoffreiche Strom enthält vorzugsweise mindestens 70 mol-% Wasserstoff, weiter bevorzugt mindestens 80 mol-% Wasserstoff, weiter bevorzugt mindestens 90 mol-% Wasserstoff, weiter bevorzugt mindestens 95 mol-% Wasserstoff, weiter bevorzugt mindestens 99 mol-% Wasserstoff.

Die Erfindung wird im Folgenden durch ein Ausführungsbeispiel näher erläutert, ohne den Gegenstand der Erfindung dadurch zu beschränken.

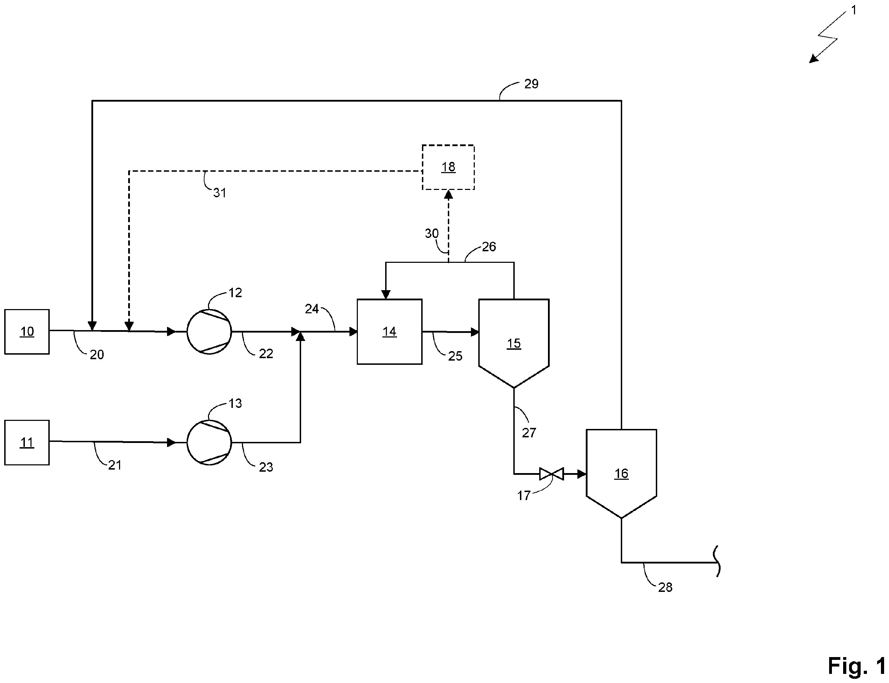

Es zeigt
- Figur 1: ein stark vereinfachtes Verfahrensfließbild eines Beispiels des erfindungsgemäßen Verfahrens zum Herstellen von Methanol.

Aus einer Wasserstoffquelle 10, beispielsweise einer Elektrolyseanlage, wird ein erster wasserstoffreicher Eduktgasstrom bereitgestellt. Der erste Eduktgasstrom kann beispielsweise einen Wasserstoffgehalt von mehr als 99 Gew.-% aufweisen.

Aus einer Kohlendioxidquelle 11, beispielsweise einer Kohlendioxidabscheideanlage, wird ein zweiter kohlendioxidreicher Eduktgasstrom bereitgestellt. Der zweite Eduktgasstrom kann beispielsweise einen Kohlendioxidgehalt von mehr als 99 Gew.-% aufweisen.

Der erste Eduktgasstrom wird über Leitung 20 weitergeführt und mit einem Entspannungsgasstrom aus Leitung 29 zusammengeführt. Dadurch wird ein erster Mischgasstrom gebildet. Der Entspannungsgasstrom besteht im Wesentlichen aus Wasserstoff und Kohlendioxid. Optional wird der erste Mischgasstrom mit einem wasserstoffreichen Strom aus Leitung 31 an Wasserstoff angereichert. Der wasserstoffreiche Strom in Leitung 31 wird in einer Vorrichtung für Wasserstoffrückgewinnung 31 erzeugt. Alternativ dazu kann der wasserstoffreiche Strom aus Leitung 31 mit dem ersten Eduktgasstrom gemischt werden, und der an Wasserstoff angereicherte erste Eduktgasstrom wird anschließend mit dem Entspannungsgasstrom aus Leitung 29 gemischt, wodurch ein an Wasserstoff angereicherter erster Mischgasstrom erhalten wird.

Der resultierende erste Mischgasstrom wird über Leitung 20 weitergeführt und in einem Kompressor 12 auf einen für die Methanolsynthese geeigneten Synthesedruck verdichtet.

Der Druck in Leitung 29 ist höher als der Druck in Leitung 20. Aus diesem Grund wird für das Einspeisen des Entspannungsgasstroms aus Leitung 29 in Leitung 20 kein in Leitung 29 angeordneter Kompressor benötigt.

Der zweite Eduktgasstrom wird über Leitung 21 weitergeführt und in Kompressor 13 auf einen für die Methanolsynthese geeigneten Druck verdichtet. Der Druck in Leitung 21 ist höher als in Leitung 20. Daher wird der Entspannungsgasstrom aus einem zweiten Abscheider 16 über Leitung 29 in Leitung 20 eingespeist. Durch den niedrigeren Druck in Leitung 20 kann der Druck im zweiten Abscheider 16, der als Niederdruckabscheider ausgestaltet ist, niedriger gewählt werden, so dass aus dem zweiten Abscheider 16 eine möglichst große Menge an Entspannungsgas erhalten wird.

Der verdichtete erste Mischgasstrom wird über Leitung 22 weitergeführt, und der verdichtete zweite Eduktgasstrom wird über Leitung 23 weitergeführt. Beide vorgenannten Ströme werden anschließend in Leitung 24 zusammengeführt, wodurch ein für die Methanolsynthese geeigneter Synthesegasstrom in Leitung 24 erhalten wird.

Aufgrund der getrennten Verdichtung der Ströme in Leitung 20 und 21 ist eine Rückführung des Entspannungsgasstroms aus Leitung 29 zu dem bereits gemischten Synthesegasstrom nicht möglich. Erfindungsgemäß wird der Entspannungsgasstrom aus Leitung 29 daher dem noch nicht verdichteten ersten Eduktgasstrom in Leitung 20 zugeführt, um den ersten Mischgasstrom zu bilden.

Der auf Synthesedruck verdichtete Synthesegasstrom wird über Leitung 24 in eine Reaktionsvorrichtung 14 eingeleitet, welche mindestens einen Methanolsynthese-Reaktor mit einem geeigneten Festbett mit einem Methanolsynthese-Katalysator umfasst. Bei dem Methanolsynthese-Katalysator kann es sich um jeden dem Fachmann bekannten geeigneten Katalysator, beispielsweise auf Kupfer-Basis, handeln.

In der Reaktionsvorrichtung 14 wird der Synthesegasstrom in einer exothermen Gleichgewichtsreaktion zu einem methanolhaltigen Produktgasgemisch umgesetzt, welches Methanol und Wasser, sowie nicht zu vermeidende Nebenprodukte enthält. Der erhaltene Produktgasstrom wird anschließend unter den Taupunkt von Methanol beziehungsweise Wasser gekühlt, um hauptsächlich Methanol, Wasser und kondensierbare Nebenprodukte als Rohmethanolstrom zu kondensieren. Die dafür erforderlichen Vorrichtungen (Kühler, Kondensator) sind nicht gezeigt. Der zweiphasige Strom, welcher außer den kondensierten Bestandteilen auch nicht umgesetztes Synthesegas enthält, wird über Leitung 25 in einen ersten Abscheider 15 eingeleitet, welcher als Hochdruckabscheider ausgestaltet ist.

Im ersten Abscheider 15 wird die flüssige von der gasförmigen Phase getrennt. Die gasförmige Phase besteht weitgehend aus in der Reaktionsvorrichtung 14 nicht umgesetztem Synthesegas, welches über Leitung 26 aus dem ersten Abscheider ausgeleitet wird und nach erneuter Verdichtung auf Synthesedruck mittels eines innerhalb der Leitung 26 angeordneten Kompressors (nicht gezeigt) zu einem Eingang (nicht gezeigt) der Reaktionsvorrichtung 14 zurückgeführt wird, um weiter zu Methanol umgesetzt zu werden. Das Gasgemisch in Leitung 26 wird auch als Kreislaufgas bezeichnet. Ein Teil dieses Kreislaufgases wird über Leitung 30 als Purge-Gas abgezweigt, um die Anreicherung von inerten Bestandteilen im Methanolsynthese-Kreislauf zu verhindern. Das Purge-Gas in Leitung 30 wird der Vorrichtung zur Wasserstoffrückgewinnung 18 zugeführt, um aus dem Purge-Gas einen an Wasserstoff angereicherten Strom zu gewinnen. Bei der Vorrichtung zur Wasserstoff-Rückgewinnung kann es sich beispielsweise um eine Vorrichtung handeln, welche einen reinen Wasserstoffstrom nach dem Prinzip der Wechseldruckadsorption (PSA) Prinzip erzeugt, oder um eine Membran, die einen an Wasserstoff angereicherten, kohlenstoffdioxidhaltigen Strom erzeugt.

Aus dem ersten Abscheider 15 wird weiterhin über Leitung 27 ein erster Rohmethanolstrom ausgeleitet, der über ein Druckminderventil 17 druckentspannt wird und anschließend in den zweiten Abscheider 16 eingeleitet wird. Der Druck im zweiten Abscheider 16 wird so gewählt, dass er etwas höher ist als der Druck in der den ersten Eduktgasstrom führenden Leitung 20.

Aus dem zweiten Abscheider 16 wird auch ein zweiter Rohmethanolstrom über Leitung 28 ausgeleitet. Dieser Rohmethanolstrom wird einer weiteren Aufarbeitung zur Gewinnung von Reinmethanol, beispielsweise einer thermischen Trennvorrichtung (Destillation) zugeführt (nicht gezeigt).

Der Entspannungsgasstrom enthält in etwa 0,4 % der über den ersten Eduktgasstrom zugeführten Wasserstoffmenge. Ferner enthält der Entspannungsgasstrom in etwa 1,1 % der über den zweiten Eduktgasstrom zugeführten Kohlendioxidmenge. Entsprechend wird der Verbrauch an Wasserstoff- und Kohlendioxid-Edukt verringert, was in einer entsprechenden Absenkung der Kohlendioxidemissionen resultiert sowie in einem geringeren Gesamtenergiebedarf zur Erzeugung des Wasserstoffs mittels Elektrolyse.

### Bezugszeichenliste

- 1: Erfindungsgemäßes Verfahren
- 10: Wasserstoffquelle
- 11: Kohlendioxidquelle
- 12, 13: Kompressor
- 14: Reaktionsvorrichtung
- 15: Erster Abscheider (Hochdruckabscheider)
- 16: Zweiter Abscheider (Niederdruckabscheider)
- 17: Druckminderventil
- 18: Vorrichtung zur Wasserstoffrückgewinnung
- 20-31: Leitung

## Patentansprüche

1. Verfahren zur Herstellung von Methanol, wobei das Verfahren folgende Verfahrensschritte umfasst:
(a) Bereitstellen eines ersten Eduktgasstroms, welcher reich an Wasserstoff (H₂) ist;
(b) Bereitstellen eines zweiten Eduktgasstroms, welcher reich an Kohlendioxid (CO₂) ist;
(c) Bereitstellen eines Entspannungsgasstroms, welcher Wasserstoff und Kohlendioxid enthält;
(d1) Zusammenführen des Entspannungsgasstroms mit dem ersten Eduktgasstrom vor Verdichtung auf Synthesedruck zum Bilden eines ersten Mischgasstroms, und Bilden eines Synthesegasstroms durch Zusammenführen des ersten Mischgasstroms mit dem zweiten Eduktgasstrom, oder
(d2) Zusammenführen des Entspannungsgasstroms mit dem zweiten Eduktgasstrom vor Verdichtung auf Synthesedruck zum Bilden eines zweiten Mischgasstroms, und Bilden eines Synthesegasstroms durch Zusammenführen des ersten Eduktgasstroms mit dem zweiten Mischgasstroms, oder
(d3) Aufteilen des Entspannungsgasstroms in einen ersten Teilstrom und einen zweiten Teilstrom, und Zusammenführen des ersten Teilstroms mit dem ersten Eduktgasstrom vor Verdichtung auf Synthesedruck zum Bilden eines dritten Mischgasstroms, und Zusammenführen des zweiten Teilstroms mit dem zweiten Eduktgasstrom vor Verdichtung auf Synthesedruck zum Bilden eines vierten Mischgasstroms, und Bilden eines Synthesegasstroms durch Zusammenführen des dritten Mischgasstroms mit dem vierten Mischgasstrom;
(e) Umsetzen des Synthesegasstroms bei Synthesedruck in einer Reaktionsvorrichtung an einem festen Methanolsynthese-Katalysator zu einem methanolhaltigen Produktgasstrom;
(f) Abtrennen von Rohmethanol aus dem Produktgasstrom in einem ersten Abscheider, wobei ein erster Rohmethanolstrom als flüssige Phase und ein Strom aus nicht reagiertem Synthesegas als gasförmige Phase erhalten werden, und wobei zumindest ein Teil des Stroms aus nicht reagiertem Synthesegas zu einem Einlass der Reaktionsvorrichtung zurückgeführt wird, und der erste Rohmethanolstrom aus dem ersten Abscheider ausgeleitet wird;
(g) Einleiten des ersten Rohmethanolstroms in einen zweiten Abscheider, wobei der Druck im zweiten Abscheider gegenüber dem Druck im ersten Abscheider vermindert ist, wodurch im zweiten Abscheider ein zweiter Rohmethanolstrom als flüssige Phase und der Entspannungsgasstrom als gasförmige Phase erhalten werden, wobei der zweite Rohmethanolstrom und der Entspannungsgasstrom aus dem zweiten Abscheider ausgeleitet werden, und wobei der Entspannungsgasstrom durch das Ausleiten aus dem zweiten Abscheider bereitgestellt wird.

2. Verfahren nach Anspruch 1, **dadurch gekennzeichnet, dass**
(h1) der erste Mischgasstrom und der zweite Eduktgasstrom getrennt voneinander auf Synthesedruck verdichtet werden, und nach Verdichtung auf Synthesedruck zusammengeführt werden, um den Synthesegasstrom zu bilden, oder
(h2) dass der erste Eduktgasstrom und der zweite Mischgasstrom getrennt voneinander auf Synthesedruck verdichtet werden, und nach Verdichtung auf Synthesedruck zusammengeführt werden, um den Synthesegasstrom zu bilden, oder
(h3) dass der dritte Mischgasstrom und der vierte Mischgasstrom getrennt voneinander auf Synthesedruck verdichtet werden, und nach Verdichtung auf Synthesedruck zusammengeführt werden, um den Synthesegasstrom zu bilden.

3. Verfahren nach Anspruch 1 oder 2, **dadurch gekennzeichnet, dass** der erste Eduktgasstrom und der zweite Eduktgasstrom unterschiedliche Drücke aufweisen, und dass der Entspannungsgasstrom mit demjenigen Eduktgasstrom zusammengeführt wird, welcher den niedrigeren Druck gegenüber dem anderen Eduktgasstrom aufweist.

4. Verfahren nach Anspruch 3, **dadurch gekennzeichnet, dass** der Entspannungsgasstrom einen höheren Druck aufweist als der Eduktgasstrom mit dem gegenüber dem anderen Eduktgasstrom niedrigeren Druck, und dass der Entspannungsgasstrom vor dem Zusammenführen mit dem demjenigen Eduktgasstrom, welcher gegenüber dem anderen Eduktgasstrom einen niedrigeren Druck aufweist, nicht verdichtet wird.

5. Verfahren nach einem der vorhergehenden Ansprüche, **dadurch gekennzeichnet, dass** der zweite Eduktgasstrom, der zweite Mischgasstrom oder der vierte Mischgasstrom vor dem Verdichten auf Synthesedruck einem Reinigungsverfahren unterzogen wird, insbesondere einem Reinigungsverfahren zur Entfernung von schwefelhaltigen Verbindungen oder zur Entfernung von Sauerstoff unterzogen wird.

6. Verfahren nach Anspruch 5, **dadurch gekennzeichnet, dass** der im Entspannungsgas enthaltene Wasserstoff zur Reduktion von im zweiten Eduktgasstrom enthaltenen Verunreinigungen genutzt wird.

7. Verfahren nach einem der vorhergehenden Ansprüche, **dadurch gekennzeichnet, dass**
- der erste Eduktgasstrom mindestens 50 mol-% Wasserstoff enthält, vorzugsweise mindestens 70 mol-% Wasserstoff enthält, weiter bevorzugt mindestens 80 mol-% Wasserstoff enthält, weiter bevorzugt mindestens 90 mol-% Wasserstoff enthält, weiter bevorzugt mindestens 95 mol-% Wasserstoff enthält, weiter bevorzugt mindestens 99 mol-% Wasserstoff enthält, und
- der zweite Eduktgasstrom mindestens 50 mol-% Kohlendioxid enthält, vorzugsweise mindestens 70 mol-% Kohlendioxid enthält, weiter bevorzugt mindestens 80 mol-% Kohlendioxid enthält, weiter bevorzugt mindestens 90 mol-% Kohlendioxid enthält, weiter bevorzugt mindestens 95 mol-% Kohlendioxid enthält, weiter bevorzugt mindestens 99 mol-% Kohlendioxid enthält.

8. Verfahren nach einem der vorhergehenden Ansprüche, **dadurch gekennzeichnet, dass**
- der erste Eduktgasstrom einen Druck von 1 bar bis 90 bar aufweist, vorzugsweise einen Druck von 1 bis 50 bar aufweist, weiter bevorzugt einen Druck von 1 bis 40 bar aufweist, und
- der zweite Eduktgasstrom einen Druck von 1 bar bis 90 bar aufweist, vorzugweise einen Druck von 1 bar bis 50 bar aufweist, weiter bevorzugt einen Druck von 1 bis 40 bar aufweist.

9. Verfahren nach einem der vorhergehenden Ansprüche, **dadurch gekennzeichnet, dass** der erste Abscheider einen Behälterinnendruck von 40 bar bis 100 bar aufweist, vorzugsweise einen Behälterinnendruck von 75 bar bis 90 bar aufweist.

10. Verfahren nach einem der vorhergehenden Ansprüche, **dadurch gekennzeichnet, dass** der zweite Abscheider einen Behälterinnendruck von 1 bar bis 50 bar aufweist, vorzugsweise einen Behälterinnendruck von 5 bar bis 35 bar aufweist.

11. Verfahren nach einem der vorhergehenden Ansprüche, **dadurch gekennzeichnet, dass** der gemäß Schritt (g) aus dem zweiten Abscheider ausgeleitete Entspannungsgasstrom einem Gasanreicherungsverfahren unterzogen wird, wodurch ein Entspannungsgasstrom erhalten wird, in welchem die Wasserstoffkonzentration und/oder die Kohlendioxidkonzentration gegenüber dem aus dem zweiten Abscheider ausgeleiteten Entspannungsgasstrom erhöht ist, und wobei der so erhaltene an Wasserstoff und/oder Kohlendioxid angereicherte Entspannungsgasstrom als Entspannungsgasstrom gemäß Schritt (c) bereitgestellt wird.

12. Verfahren nach einem der vorhergehenden Ansprüche, **dadurch gekennzeichnet, dass** von dem Strom aus nicht reagiertem Synthesegas ein Teilstrom als Purge-Gas-Strom abgezweigt wird, und der Purge-Gas-Strom einer Wasserstoff-Rückgewinnungseinheit zur Erzeugung eines wasserstoffreichen Stroms zugeführt wird, und wobei zumindest ein Strom aus der Gruppe erster Eduktgasstrom, zweiter Eduktgasstrom, erster Mischgasstrom, zweiter Mischgasstrom, dritter Mischgasstrom, vierter Mischgasstrom, Entspannungsgasstrom vor Verdichten auf Synthesedruck durch Zuführen von Wasserstoff aus dem wasserstoffreichen Strom an Wasserstoff angereichert wird.
